(19) **Europäisches Patentamt / European Patent Office / Office européen des brevets**

(11) **EP 3 911 236 B1**

(12) **EUROPEAN PATENT SPECIFICATION**

(45) Date of publication and mention
of the grant of the patent:
**11.01.2023 Bulletin 2023/02**

(21) Application number: **20808448.3**

(22) Date of filing: **25.11.2020**

(51) International Patent Classification (IPC):
*A61B 6/00* $^{(2006.01)}$    *H01J 35/14* $^{(2006.01)}$
*H01J 35/24* $^{(2006.01)}$    *G01N 23/041* $^{(2018.01)}$
*G01N 23/20066* $^{(2018.01)}$    *G01N 23/201* $^{(2018.01)}$
*G01N 23/207* $^{(2018.01)}$    *H05G 1/00* $^{(2006.01)}$

(52) Cooperative Patent Classification (CPC):
**A61B 6/483; A61B 6/4021; A61B 6/4291;**
**A61B 6/4417; A61B 6/484; A61B 6/5229;**
**A61B 6/5282; G01N 23/041; G01N 23/20083;**
G01N 2223/051; G01N 2223/063; G01N 2223/419

(86) International application number:
**PCT/EP2020/083265**

(87) International publication number:
**WO 2021/110494 (10.06.2021 Gazette 2021/23)**

(54) **ESTIMATION OF FULL-FIELD SCATTERING FOR DAX IMAGING**

SCHÄTZUNG DER VOLLFELDSTREUUNG FÜR DAX-BILDGEBUNG

ESTIMATION DE DIFFUSION PLEIN CHAMP POUR IMAGERIE DAX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.12.2019 EP 19213803**

(43) Date of publication of application:
**24.11.2021 Bulletin 2021/47**

(73) Proprietor: **Koninklijke Philips N.V.**
**5656 AG Eindhoven (NL)**

(72) Inventors:
• **PREVRHAL, Sven, Peter**
**5656 AE Eindhoven (NL)**
• **KOEHLER, Thomas**
**5656 AE Eindhoven (NL)**
• **ENGEL, Klaus, Juergen**
**5656 AE Eindhoven (NL)**

(74) Representative: **Philips Intellectual Property & Standards**
**High Tech Campus 52**
**5656 AG Eindhoven (NL)**

(56) References cited:
**US-A1- 2009 232 272    US-A1- 2017 284 948**

**Description**

FIELD OF THE INVENTION

The invention relates to an X-ray imaging system configured for phase contrast and/or dark-field imaging, to a method of operating an X-ray imaging apparatus; to a computer program element and to the computer readable medium.

BACKGROUND OF THE INVENTION

Dark-field ("DAX") imaging has attracted interest especially in the medical field. Dark-field imaging is a type of X-ray imaging. Contrast in dark-field imaging relates to the amount of small angle scattering experienced by the X-radiation.

Experimental dark-field imaging with mice have been reported by A. Yaroshenko et al in "Pulmonary Emphysema Diagnosis with a Preclinical Small-Animal X-ray Dark-Field Scatter-Contrast Scanner", Radiology, vol. 269, No 2, November 2013. Phase-contrast has also been found to add additional useful insights, in particular when imaging soft-tissue.

It has been overserved that at times dark-field or phase-contrast images are sometimes corrupted by artifacts.

Document US2017284948 A1 discloses a phase contrast imaging system which uses a scatterer inserted in the beam to suppress a Moiré fringe to allow an image capturing mode with absorption imaging without removing a grating from the system.

SUMMARY OF THE INVENTION

There may therefore be a need for alternative system or method to improve dark-field or phase-contrast imaging.

The object of the present invention is solved by the subject matter of the independent claims where further embodiments are incorporated in the dependent claims. It should be noted that the following described aspect of the invention equally applies to the method of operating an X-ray imaging apparatus, to the computer program element and to the computer readable medium.

According to a first aspect of the invention there is provided an X-ray imaging system configured for phase contrast and/or dark-field imaging, comprising:

- an X-ray source operable to cause X-radiation to emanate from a focal spot of the source;
- an X-ray sensitive detector operable to detect the X-radiation after interaction of said X-radiation with an object to be imaged, if present, between the X-ray source and the detector;
- a control logic operable to cause the X-ray imaging apparatus to operate in any one of two modes, an object image acquisition mode and a scattering measurement mode, wherein, when in scattering measurement mode, the X-radiation receivable at the detector comprises a higher proportion of scattered radiation than X-radiation receivable when the system is in object image acquisition mode, and
- a scattering corrector operable, based on the measured scattering data, to reduce, or facilitate to reduce, or to prevent, a scattering artifact in an object image generatable from data acquired in image acquisition mode.

The system improves DAX and/or phase contrast image quality.

In one embodiment, the system comprises a scattering measurement facilitator arranged between the X-ray source and the detector, at least a part or parts of the scattering measurement facilitator capable of blocking X-radiation, the control logic causing, when the system is in scattering measurement mode, a change of pose of the scattering measurement facilitator relative to a pose of the scattering measurement facilitator when the system is in object image acquisition mode, so that at least the said part or parts block(s) at least some of the X-radiation. Preferably, mostly, or only, secondary radiation reaches the detector.

In one embodiment, the scattering measurement facilitator comprises any one or more of: i) an anti-scatter grid, ii) at least a part of an interferometer, iii) a code aperture plate.

In one embodiment, a direction of the X-radiation is changeable through a source interface, wherein, the control logic causes through said interface X-radiation to emanate along a first direction when the system is in object image acquisition mode and cause the X-radiation to emanate along a second direction, different from the first direction, when the system is in scattering measurement mode.

In one embodiment, the scattering measurement facilitator comprises at least a part of the interferometer, the control logic, when in scattering measurement mode, causing the change of pose of the said interferometer, or the part thereof, so as to reduce a fringe pattern detectable at the detector.

In one embodiment, the X-ray imaging system is a full-view imaging system. According to a further aspect of the invention there is provided a method comprising the steps of:

- causing an X-ray imaging apparatus configured for phase contrast and/or dark-field imaging to operate in any one of two modes, an object image acquisition mode and a scattering measurement mode, wherein, when in scattering measurement mode, the X-radiation receivable at a detector of the apparatus comprises a higher proportion of scattering radiation than X-radiation receivable when the system is in object image acquisition mode; and
- based on the measured scattering data, reducing, or facilitating reducing, or preventing, a scattering artifact in an object image generatable from data acquired in image acquisition mode.

According to a further aspect of the invention there is provided a computer program element, which, when being executed by at least one processing unit, is adapted to cause the processing unit to perform the method.

According to a further aspect of the invention there is provided a computer readable medium having stored thereon the program element.

The proposed system allows acquiring and generating quantitatively correct DAX images and/or phase contrast ("$\Phi$"-) images. The system provides sufficiently accurate "large angle"-scattering correction. The scattering correction setup as implementable by the system is tailored to the given patient and/or patient position and is able to cope with the signal decrease in the order of the square of the distance of the patient to the detector.

Applicants have established that systematic errors due to inaccuracies in the scattering estimation can already be observed in slot-scanning DAX system, and this is likely to deteriorate further for full-field system, where scattering effects will be roughly six times larger. For example, it is expected that in full-field imaging, scattering from the lung area for example will markedly deteriorate the DAX signal. The proposed system is able to cope even with large, full-field imaging scenarios.

BRIEF DESCRIPTION OF THE DRAWINGS

Exemplary embodiments of the invention will now be described with reference to the following drawings, which are not to scale, wherein:

Fig. 1 is a schematic X-ray imaging apparatus configured for dark field and/or phase contrast imaging; and
Fig. 2 is a flow chart of a method of operating an X-ray imaging apparatus configured for phase contrast and/or dark field imaging.

DETAILED DESCRIPTION OF EMBODIMENTS

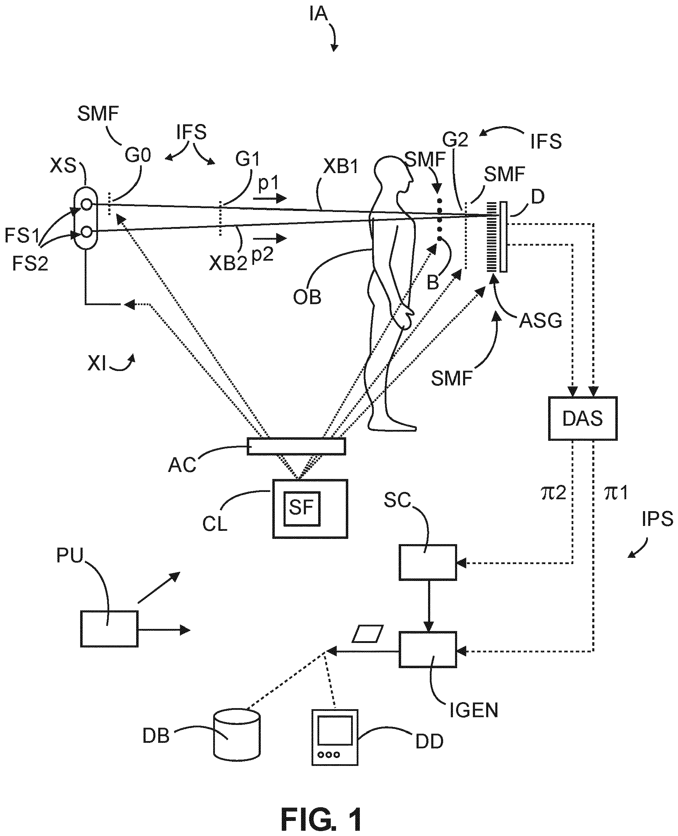

With reference to Fig. 1, there is shown a schematic block diagram of an image processing arrangement IA that includes a computerized image processing system IPS and an X-ray imaging apparatus XI ("imager"). The X-ray imaging apparatus is configured for dark-field X-ray ("DAX"-) imaging and/or phase-contrast ("$\Phi$"-) imaging. The imaging apparatus XI includes an X-ray source XR and an X-radiation sensitive detector D. The imager XI may be configured for 2D radiography imaging or for 3D imaging such as a CT scanner.

The image processing system IPS may be run as one or more software modules or routines on one or more data processing units PU such as one or more computers, servers, etc. The IPS may be arranged external to and remote of the imager XI, or the IPS is integrated into the imager XI, for instance into a work station associated with the imager XI or into an operator console. The image processing system IPS may be implemented in a distributed architecture to serve a group of imagers suitably connected in a communication network. Some or all components of the IPS may be arranged in hardware such as a suitably programmed FPGA (field-programmable-gate-array) or as hardwired IC chips. The image processing system IPS may be arranged partly in software and partly in hardware.

Broadly, the image processing system IPS includes an image generator IGEN that processes projection imagery $\pi 1$ acquired by the imager XI into dark-field and/or phase-contrast imagery. The image processing system IPS includes a scattering corrector SC to reduce scattering artifacts. The scattering corrected imagery as provided by the scattering corrector SC in cooperation with the image generator IGEN can then be displayed on a display unit DD or can be stored in a memory for later review, or can be otherwise further processed. The scattering corrector SC may be integrated into the image generator IGEN to form one functional unit. Alternatively, image generator IGEN and scattering corrector SC form separate functional units.

Although as shown in Fig. 1 the imaging apparatus XI supplies the projection imagery $\pi 1$ directly, via wireless or a wired connection, to the image processing system IPS, this may not be so in all embodiments. For instance, the projection imagery $\pi$ may be first stored in a memory such as a picture archiving system (PACS) of a hospital information system (HIS) or otherwise, and the imagery is retrieved at a later stage (e.g. upon user request) by the IPS and is then processed.

In general, the imaging apparatus XI includes a DAX/$\Phi$-imaging facilitator structure IFS. In embodiments, the DAX/$\Phi$-imaging facilitator structure includes an interferometer G0,G1,G2. The interferometer GO,G1,G2 includes one, two or three gratings. In embodiments, three gratings are used: two absorber gratings G0,G2 and a phase grating G1, to be described more fully below. In general, the DAX/Φ-imaging facilitator structure is a device, or group of devices, that allows translating X-ray beam refraction and/or small angle scattering of the beam into intensity modulations detectable at the X-ray sensitive detector D, thereby facilitating resolving said modulations into dark-field and/or phase-contrast image signals and, if desired, into an attenuation image signal.

In the following main reference will be made to an interferometric imaging apparatus although this is not at the exclusion of other grating-based or non-interferometric imaging DAX/Φ-facilitator structures. Other, non-interferometric imaging DAX/Φ-facilitator structures include coded aperture systems, crystals, or non-grating based structures, with periodic or non-periodic sub-structures.

In general, the dark-field or phase-contrast is obtainable by the DAX/Φ-imaging facilitator structure IFS imparting a periodic wave front modulation on the incoming imaging X-ray beam and by a measuring, with the X-ray detector D, a variation of the resulting wave-front caused be the object OB to be imaged.

In more detail, and referring now to the (non-limiting) interferometric embodiment, between the X-ray source XR and the detector D there is defined an imaging region where the object OB (e.g., the chest of the subject) to be imaged resides during imaging. In the imaging region there is arranged the single, two or three (or more) grating structures of the interferometer.

With continued reference to the (non-limiting) interferometric embodiment of the image facilitator structure DAX/Φ-IFS. periodicity, aspect ratio, etc. of the gratings are such that they cause diffraction of the X-ray beam and/or just enough coherence is achieved so that the small-angle scattering can be detected or derived. Absorption and phase gratings may be used. In one embodiment the gratings are formed by photolithography or cutting in silicon wafers to define a periodic pattern of trenches. Interspaces between the trenches of the absorption gratings GO,G1 may be filled with lead or gold to form respective sets of absorbing lamellae. In Fig. 1, the longitudinal axes of the grating lamella run perpendicular into the plane of the drawing.

In yet more detail, and in one embodiment, an absorbing grating structure G2 is arranged between the detector D and the object OB whilst the other grating G1, the phase grating, is arranged between the object OB and the X-ray detector D. In some embodiments there is also an additional grating G0 arranged at the X-ray source XR, in case the X-ray source is incapable of generating natively coherent radiation. If the X-ray source produces incoherent radiation (which is usually the case), the absorption grating G0 at the X-ray source (also referred to as the source grating) transforms the X-radiation coming out of the X-ray source into an, at least partly, coherent radiation beam XB. Inverse geometries where the G1 is placed upstream the object OB, i.e. between XR and OB, are also envisaged. The interferometer is in embodiments of the Talbot-Lau type. The interferometer must be precisely adjusted with grating lamellae aligned. The distance between G1 and G2 is a suitable Talbot distance. When properly adjusted, fringe patterns can be detected at the detector D when exposed to X-radiation. Based on the fringe pattern, the DAX/Φ imagery can be generated by the image generator IGEN.

The interferometer G0, G1, G2 is preferably focused. In other words, one, two or all three of the gratings G0, G1, G2 have their lamella focused on the focal spot FS1. In some such focused embodiments, the gratings are curved to form respective lateral surfaces of three imaginary, concentric cylinders. A central axis of these imaginary cylinders passes through the focal spot FS1 when properly focused. In particular, the absorption gratings G1 and G2 are curved and/or focused. If the DAX/Φ-facilitator structure is not an interferometer, this too may be focused on the focal spot FS1.

The at least partly coherent radiation beam XB propagates through the imaging region and interacts with the interferometer gratings G1,G2 and the patient OB. After said interaction, the radiation is then detected in form of electrical signals at radiation sensitive pixel elements of the detector D. Data acquisition circuitry DAS digitalizes the electrical signals into projection (raw) image data π1, which is then processed by the IPS in a manner explained in more detail below.

The imaging apparatus XI may be of the full field-of-view (FoV) type, where the detector is of the flat panel type. In full FoV imaging system, the size of the detector D and the size of the IFS corresponds to the desired FoV. Alternatively, the detector D and the imaging facilitator structure IFS may be smaller than the intended FoV such as in slot-scanning systems as shown in Fig 1. In some of these systems, the detector comprises a discrete series of detector lines. The detector lines are mounted on a scan arm to be scanned across the intended FoV in different slot-positions. The absorber grating G2 is generally coextensive with the detector in full field-of-view (FoV) type imagers.

Slot-scanning systems as shown in Fig. 1 are more cost effective than full FoV system because they require smaller detectors and smaller gratings IFS. The gratings IFS are mounted on the scan arm above the detector and are equally scanned across the FoV. In alternative slot-scanning systems, although the detector D has the same size as the desired FoV, the gratings are smaller and a collimation SC is used to scan only parts of the FoV (in "slots") at any one time as per the collimation. In a full FoV system and slot scanning systems with a non-moving flat panel detector, there is a simple one-to-one relationship between pixel position and imaginary geometrical rays that pass through the imaging region to define the imaging geometry. The rays extend from a focal spot of the X-ray source XR and intersect the detector plane at the respective pixel positions. Each one of the geometrical rays corresponds to a respective, different, single one of the pixels. No such simple relationship exists in some slot-scanning system with smaller detector, where each geometrical ray is seen by many different pixels in different "slots" during the scanning. Signals from different pixels are then processed together by a suitable logic for any single geometrical ray.

The image generator IGEN outputs the dark-field signals and/or the phase-contrast signals as respective arrays of image values which form the dark-field image and the phase-contrast image, respectively. These image values or pixel values represent respectively the contrast for the dark-field signal and the phase-change experienced by the X-radiation while traveling through the object OB, for the respective geometrical ray.

Generally, when X-radiation interacts with material, it experiences both attenuation and refraction and hence phase change. The attenuation on the other hand can be broken down into attenuation that stems from photo-electric absorption and attenuation that stems from scatter. The scattering contribution in turn can be decomposed into Compton scattering and Rayleigh scattering. For present purposes of dark-field imaging it is the small angle scattering that is of interest, where the qualifier *"small angle"* means that the scatter angle is so small that the scattered photon still reaches the same pixel as it would have reached had it not been scattered at all.

The dark-field contribution can be modelled as visibility $V = V_0 * e^{-\int \varepsilon(z)dz}$, with $\varepsilon$ is the spatial distribution of the diffusion property of the patient OB and the integration is performed along the X-ray beam path, and $V_0$ being the reference visibility without object interaction (recorded in a calibration measurement). The dark-field signal as recorded in the dark-field image is then $D = V/V_0$.

Traditional radiography systems are usually incapable of resolving the detected signal into dark-field contribution. But by using the interferometer GO.G1,G2 as shown in Fig. 1, or by using other DAX/$\Phi$ imaging facilitator structures IFS, it is possible to translate these contributions into an intensity pattern of fringes which can be analyzed by the image generator IGEN to obtain the phase-contrast and/or DAX image. The image generator IGEN acts on a series of projection images obtained in a phase stepping operation. Based on this recorded series of projection images $\pi1$, the image generator IGEN computationally resolves the detected fringe pattern in the series projection data $\pi1$ into three contributions or signals, namely the refraction contribution (also referred to as the phase-contrast signal), the dark-field signal component and a remaining attenuation component. Because of these three contrast mechanisms acting together, the signal processing by the IGEN of the detected series of intensities proceeds in three signal channels (phase-contrast, dark-field, and attenuation).

In the above described types of imaging system, the capability for dark-field/phase-contrast imaging is achieved as follows: the projection data is acquired at the detector D during the phase stepping operation (where the phase of the fringes is stepped typically over 360°) as a series for a given fixed projection direction. The phase stepping operation is typically realized by inducing a motion between X-ray beam and one of the gratings of the image facilitator structure IFS (if an interferometer is used). For instance, in one embodiment the analyzer grating G1 is moved ("scanned") laterally relative to an optical axis of the X-ray beam. Alternatively, phase stepping can also be achieved by moving the patient OB as in Fig. 1, or by moving the X-ray source, etc. This motion causes a change of the fringe pattern which in turn can be recorded in the corresponding series for each step of the motion thus resulting in phase stepping. This series of measurements form, for each geometrical ray, an associated phase curve. The phase curves are in general of sinusoidal shape and it has been found that each encodes the quantities of interest, in particular the dark-field signal, along with attenuation and phase change.

In yet more detail, the phase curves for each pixel/geometrical ray can be respectively analyzed, for instance by fitting to a sinusoidal signal model to effect the image generation. Preferably, there are at least three fitting parameters included in the three-channel sinusoidal model. The three fitting parameters represent, respectively, the three contributions phase-contrast, dark-field signal and attenuation. The sinusoidal model is fitted by image generator IGEN to the phase curves to so compute in particular the DAX and/or $\Phi$ image, and an attenuation (also called "transmission") image, although this is of lesser interest herein. Computation of the apparently superfluous transmission image may be required to correctly account for the three contrast effects as otherwise incorrect contributions are incurred in the DAX and/or $\Phi$-channel.

An optimization procedure is used to fit the measured series of projections to the model. The procedure can be understood in terms of as cost function and the fitting operation can be formulated as an optimization problem. Any suitable optimization scheme such as gradient descent, conjugate gradients, Newton-Raphson, stochastic gradients, Maximum Likelihood approach, other statistical techniques or others are also envisaged. Non analytical methods such as neural networks or other Machine Learning techniques may also be used.

In general, an optimization problem for a signal model S has the following structure:

$$\mathrm{argmin}_{T,D,\varphi}\, F = ||\, \pi - S^{T,D,\varphi}\, (X)\, || \qquad (1)$$

wherein $S^{T,D,\varphi}$ (.) is an at least three-channel modulator function that describes how the three contrast mechanism combine to modulate and transform the incoming (undisturbed) radiation $X$ into the measured data $\pi$, and $|| \cdot ||$ is a suitable similarity measure, a $p$-norm for instance, (squared) Euclidean distance, etc. Function $F$, the objective function (in this case a cost function), measures how well the signal model $S$ "explains", or "fits", the measured data $\pi$. The optimization task is how best to choose the parameters ($T$, $D$, $\phi$) of the model, with the similarity measure $\| . \|$ quantifying the goodness of fit. The function $F$ is thus a cost or error function. The task in the optimization is to improve the cost function by adjusting parameters ($T$, $D$, $\phi$). In this embodiment, the parameters are to be adjusted in the optimization so that the "cost" returned by the cost function $F$ decreases. More than three channels may be used in signal model $S$, depending on the number of contrast mechanism one wishes to account for. In (1), $F$ may be a function of residues (terms). Each residue quantifies a respective deviation, or cost, of given projection image from its prediction under model $S$.

More specially, in one embodiment, as a special case of (1), the following analytical signal model $F = \Delta^2$ optimization is used per pixel or geometrical ray:

$$\Delta^2(T, D, \phi) = \sum_i w_i (M_i - I_i T (1 + V_i D \cos(\phi - \alpha_i)))^2 \qquad (2)$$

wherein $M_i$ are the measured data (taken from $\pi$), the undisturbed radiation "X" is represented by $I_i$, $V_i$, and $\alpha_i$, the blank scan intensities, blank scan visibilities, and blank scan phases, respectively. $T$, $D$, and $\phi$ are the three contrast modulators of $S()$ above namely: transmission, dark-field, and differential phase for this image point. $w_i$ are optional statistical weights, usually selected to be equal or proportional to the inverse variance of the measured data $M_i$, and $i$ indicates the phase steps. Following on from (1), the task in (2) is to minimize the cost $\Delta^2$ over the measured data $M_i$ to find in particular the images $D$ and $\phi$. The right hand side of (2) may be understood as a weighted sum of the residues.

The above described types of image generation algorithms (1),(2) are sometimes referred to as "phase retrieval" but this is for present purposes a misnomer as there is also the dark-field image co-generated in the fitting operation and so is in fact the transmission image as mentioned above. A phase retrieval algorithm is described in Pfeiffer et al in "Hard-X-ray dark-field imaging using a grating interferometer", published in Nature Materials 7, pp 134-137 (2008). Other phase retrieval algorithms, Fourier-based or not, are also envisaged in embodiments.

The DAX/$\phi$ X-ray imaging system XI as proposed herein is configured to reduce, or to entirely eliminate, unfavorable scattering effects on an imaging operation. In particular, combating effects of large angle scattering, collectively referred to herein as "secondary radiation", is advantageous in DAX imaging as preferably envisaged herein although scattering management is also advantageous for $\phi$-imaging. Large angle scattering management is advantageous for DAX imaging as in this imaging modality one wishes to exploit small angle scattering as a contrast mechanism. Following up on the definition of "small angle" scattering given above, "large angle" scattering as used herein is the complement of small angle. Specifically, large angle relates to scattering angles large enough so that the scattered photon no longer reaches the same pixel as it otherwise would have reached, had it not been scattered at all or had it been scattered by small angle only. In this connection, "primary radiation" includes only (or predominantly) radiation modulated only by absorption, phase contrast and the desired small angle scattering. Secondary radiation is predominantly modulated by large angle scattering.

So that these two effects, large angle and small angle scattering, are not confounded, it is envisaged herein to reduce secondary radiation effects on the imagery obtainable by the proposed imaging system IPS. The proposed approach is a two pronged approach in embodiments.

In one prong, it is envisaged to provide the imager XI with an anti-scatter grid ASG to separate primary radiation from secondary radiation. In the second prong, the opposite is done, namely secondary radiation is separated from primary radiation to measure the amount of secondary radiation for the given object PAT one wishes to image. This measurement is then used to inform DAX-image generation by generator IGEN to compensate, remove, reduce, or suppress secondary radiation image artifacts.

Turning now initially to the first prong, the ASG includes a set of lamella made from a high density material such as lead or other high Z-material. A longitudinal direction of the lamella extends into the drawing plane of the schematic drawing in Fig. 1. They preferably run the full length of one spatial dimension of the detector whilst a number of lamella is chosen to essentially cover the other spatial dimension. In other words, the ASG is co-extensive with a detector surface of the detector D. The lamella are preferably, but not necessarily in all embodiments, focused on the focal spot FS of the X-ray source XS. In other words, lamellae are arranged at a larger inclination the further away they are from a center point of the detector ASG.

When focused or properly adjusted, the ASG lamella in front of the detector D block out unwanted secondary radiation. The best focus pose (that is, position and orientation) of the ASG is when the focal spot FS is situated on the imaginary line formed by a set of imaginary planes that one may pass through the respective lamellae at the given inclination of the respective lamella. The planes intersect in an imaginary line that is the central axis of the imaginary cylinders mentioned above in relation to the focused gratings G0, G1, G2. However, even in this focused arrangement, the ASG effectively blocks out only a certain fraction of secondary radiation.

In order to yet better account for substantially all, or at least a higher fraction of, secondary radiation effects, the second prong is provided. The second prong is implemented by a computerized control logic CL of the imaging system XI. The control logic causes the imaging system to assume a configuration in which a high fraction, or substantially all, of the incident, patient specific PAT secondary radiation can be measured by the detector D.

Broadly, the control logic CL is configured to allow operating the imaging system in two modes: (object) image acquisition mode and in a scattering measurement mode. The imaging system IS may include a user interface (not shown) to allow switching between scattering measurement mode and image acquisition mode. The user request for this switch may be received through touch screen action on a touch screen display, or by a pointer-device (eg, computer mouse), or any other UI input arrangement preferably provided at an operator console (not shown) of the imaging system IS.

In image acquisition mode, the DAX/$\phi$-imaging facilitator IFS and the ASG are in a focused arrangement as explained above, focused on a focal spot FS 1 of the X-ray source. In this arrangement, object imagery $\pi$I, in particular projection images, are acquired by operating the X-ray source to emanate an X-ray beam XB1 along a primary (mean) radiation direction p1 whilst the described components are aligned or focused relative to each other.

The control logic CL is configured to switch from this object acquisition mode into the scattering measurement mode where an out-of-focus (relative to focal spot FS1) is achieved by effecting a change of the imaging geometry. In this out-of-focus arrangement, again the X-ray source is energized, but preferably at low dose (lower than the dose in image acquisition mode), to acquire a second set of projection data $\pi$2, a sample, that measures the fraction of secondary radiation. Multiple embodiments on how this out-of-focus imaging geometry can be achieved are envisaged and will be explored more fully below. The control logic CL is switchable to switch the imaging system IS into each of these modes, one after the other. In this manner, respective sets of measurement data $\pi$1, $\pi$2 are measured at the detector and acquired at the data acquisition circuitry DAS and passed on to the image processing section IPS. The acquisition of the two data sets $\pi$1, $\pi$2 can be done in any order.

Preferably, however, the scattering measuring data $\pi$2 in the scattering measuring mode is acquired first in a first low dose exposure for a given patient. The measured scattering data $\pi$2 encode the specific "scattering footprint" of the given patient that reflects the patient's individual anatomic tissue composition. It is expected that this scattering measurement $\pi$2 needs to be done only once for the given patient PAT, and can be stored for later reference in a database or other memory such as a PACS in a HIS or similar. The scattering data $\pi$2 may then be retrieved for follow-up imaging appointments. Alternatively, a new up-to-date scattering measurement $\pi$2 is acquired more than once, possibly every time, when the patient is imaged, to so account for different posture/position of the patient which may also affect the secondary radiation detectable at the detector D.

The "proper" image data $\pi$1 as acquired in object image acquisition mode is received in the image processing system IPS to apply any one of the above described DAX/$\phi$ imaging processing algorithms, in particular phase retrieval algorithms, to extract the DAX or $\phi$ information to so produce respectively scattering corrected DAX and/or $\phi$ imagery of the patient. The scattering correction is achieved by the image generator IGEN interfacing with a scattering corrector component SC of the image processing system IPS. The scattering corrector component SC processes the scattering measurement data $\pi$2 in coordination with the image generator. A number of different embodiments are envisaged for this which will be described.

The measurement $\pi$2 can either be used directly as scattering estimate or may be processed first by low-pass filtering or other pre-processing. The measurement $\pi$2 (possibly so pre-processed) can serve as calibration input to scattering correction based on simulation computation of scatter. Monte Carlo methods may be used in the simulation.

In embodiments, the scattering corrector component SC and the image generator operate together on both data sets $\pi$2, $\pi$1.

Specifically, the scattering corrector CS may apply the scattering measurement $\pi$2-based correction pre-phase retrieval or post phase retrieval. For instance, the scattering data $\pi$2 may be subtracted by the corrector SC from the image data $\pi$1, and it is the difference values $| \pi1- \pi2 |$ that are then processed in the phase retrieval algorithm by the image generator IGEN. Alternatively, the DAX/$\phi$ imagery $I_{DAX}$ or $I_{\phi}$ are generated first based on $\pi$1 by the image generator IGEN, and $\pi$2 is then subtracted from $I_{DAX}$ or $I_{\phi}$ or is otherwise combined to obtain scattering corrected imagery. In general, the scattering corrector component SC uses the scattering measurement data $\pi$2 to correct the DAX and/or $\phi$ imagery for artifacts due to secondary radiation.

In other embodiments, the scattering measurement data $\pi$2 is fed by the scattering corrector component SC into the image generator IGEN and both data sets are processed together by the image generator IGEN to obtain scattering-corrected output DAX and/or $\phi$-imagery In this and similar embodiments, it may be the retrieval algorithm implemented by the image generator IGEN that is modified to include a further channel or fitting variable for the large-angle-scattering contribution into the optimization procedure of function (1) or (2) when fitting the model to the data $\pi_2$ and $\pi_1$. In this modified optimization scheme, the large angle scattering as measured by data $\pi_2$ can be accounted for in a bespoke manner for the given patient. The measured scattering data $\pi_2$ may be used in other scattering correction schemes, analytic or machine-learning based, all envisaged herein.

With continued reference to Fig. 1, different embodiments for implementing the scattering measure mode will now be explained in more detail.

'In a first embodiment, the out-of-focus configuration with respect to the focal spot FS1 of the X-ray source is obtained by changing the position of the focal spot itself from one focal spot position FS1 to another FS2, relative to the ASG as indicated in Fig. 1. The focal spot switch can be controlled by control logic CL through a suitable actuator AC. The actuator AC may be controlled by the control logic CL through an X-ray source XR interface SI.

In these embodiments, the beam XB1 propagates along a standard direction p1 when in image acquisition mode. In this configuration, the propagation direction p1 follows the focused arrangement. In the out-of-focus arrangement, caused by moving the focal spot FS 1 to another position FS2, there is a new X-ray beam XB2 propagating along a different direction p2 which is now out of focus relative to the DAX/$\phi$-imaging facilitator structure IFS and/or the ASG.

By moving the X-ray focal spot FS 1 out of the focus of the ASG, the scatter fraction can now be measured. In this out-of-focus imaging geometry, it is now primary, non-scattered radiation, that will be blocked by the ASG as the ASG is now misaligned with respect to the radiation XB2 emanating from the new focal spot FS2. The only (or at least a high fraction of) radiation left to reach the detector D is secondary radiation that is now not blocked by the ASG. Said differently, the radiation now detected will mainly consist of X-ray scattering not blocked by the ASG. This radiation, if measured with the patient in position, will thus provide an accurate estimate of the amount of non-suppressed scatter.

There are a number of different embodiments envisaged for the focal spot change. For instance, the anode and/or cathode may be shifted or otherwise have their pose changed by the actuator AC so that the X-ray beam is directed to a different direction. Alternatively, the actuator AC includes an electronic focal spot selector such an electrostatic deflection device. The electrostatic deflection device AC changes a direction of an electron beam inside the tube so that the focal spot is moved and hence the direction of the X-ray beam.

Instead of, or in addition to, changing the focal spot position in the X-ray source, other embodiments are envisaged for bringing about the out-off-ASG-focus configuration for scattering measurement mode. In these embodiments, there is a scattering measurement facilitator SMF integrated into the imaging system XI. The scattering measurement facilitator SMF is in embodiments a hardware structure arrangeable between the X-ray source XS and detector D to facilitate scattering measurements. The control logic CL disturbs or changes a pose of the scattering measurement facilitator SMF, by imparting a translation or rotation on the SMF, to so make the imaging system enter into scattering measurement mode. The scattering measurement facilitator SMF as envisaged may be able to serve different functions: in one function the SMF facilitates in scattering measurement when the system IS is in scattering measurement mode. In embodiments, the scattering measurement facilitator SMF may be switched into a configuration where it serves another imaging supporting function when the system is in image acquisition mode. In alternative embodiments, the SMF is dedicated and serves no other imaging function and is completely retired by the control logic CL when the system IS is in image acquisition mode.

In some embodiments where the scattering measurement facilitator SMF has a dual or multi-function, it is assumed that it is arranged-in-focus relative to the focal spot when the system is in image acquisition mode. But for use in scattering measurement mode, the scattering measurement facilitator SMF is moved out of focus relative to the focal spot FS1.

An actuator AC may be used to move the scattering measurement facilitator SMF out of focus. In general, the actuator setup for moving the scattering measurement facilitator SMF out of focus can be done in a number of different ways. The scattering measurement facilitator SMF may be held in a frame or other fixture movable mounted, either translatably or rotatably or both. The actuator AC, such as a gearing or screw mechanism, powered by a power source, may be used to engage with the frame to push or pull or rotate the frame, and with it the SMF, to realize the out of focus configuration. Hydraulic or piezoelectric actuators AC are also envisaged in embodiments. The actuator AC responds to control signals issued by the control logic CL through suitable device interfaces. The control logic CL in turn may answer to request signals issued by the user interface UI.

In some embodiments, the scattering measurement facilitator SMF is part of the DAX/$\phi$ imaging facilitator discussed above. For instance, in embodiments scattering measurement facilitator SMF is either one or both of the two absorption gratings G0, G2 of an interferometer, assuming the DAX/$\phi$ imaging facilitator includes such an interferometer. In this embodiment, the pose of either G0 or G2 are changed by the actuator AC. For instance, either one is rotated or shifted. Preferably, in embodiments the G2 grating is rotated by $90^0$ around the optical axis that passes through the center of the said grating G2, the axis passing through the focal spot FS1 of the X-ray source XS. It is not required that the focal spot is changeable. Rotations by angular amounts other $90°$ are also envisaged. In addition or instead of rotating the analyzer grating G2, the source G0 grating may be rotated by $90°$ (or by any other angular amounts) in either direction.

It has been observed that some residual primary radiation still reaches the detector even when either one or both of the gratings G0, G2 are moved in the above described embodiments. In this case, it may be desirable to ensure that there is no remaining fringe pattern in the data. In order to ensure this, G0 or G2 can be moved or rotated. In an exemplary embodiment, the movement of G0 or G2 to reduce the fringe pattern may be done in a feedback loop arrangement that includes the control logic CL, the actuator AC, and the detector D. The control logic CL senses, via the detector D, the fringe pattern. The control logic CL instructs the actuator AC to move G0 or G2 so as to reduce the intensity of the fringe pattern as detected at the detector D with a goal to remove or at least reduce an intensity of the fringe pattern based on suitably defined negligibility threshold. In this embodiment the CL control logic may include a machine learning component configured to implement a reinforcement learning algorithm. In this embodiment, the control logic operates the actuator to change the pose of G0 or G2. This action is driven by optimizing a reward function R that measures the intensity of the fringe pattern as detected. The readjustment of the pose of G0 or G2 is done under exposure of a preferably low x-radiation. The goal in this machine learning algorithm is to make the fringe pattern vanish as measured by the reward function. The visibility or related quantities may be used to formulate the reward function such as $R = (I_{max}-I_{min})/(I_{max}+I_{min})$, where $I_{min}$ and $I_{max}$ are the respective maximum and minimum intensities.

Although in the above embodiments it was assumed that the DAX/$\phi$ imaging facilitator is an interferometer, this is not necessarily so in all embodiments and the above described scattering measurement mode can be practiced equally in non-interferometric DAX/$\phi$ imaging setups. For example, the DAX/$\phi$ imaging facilitator may be a coded aperture plate or other and this is moved by the control logic CL and the actuator AC.

In another embodiment, the scattering measurement facilitator SMF includes the ASG itself. The actuator AC is controlled by the control logic to change a pose of the AGS. For instance, the ASG may be rotated by a certain angle, so that the longitudinal axes of its lamella point now to a different direction and no longer extended into the drawing plane as shown schematically in Fig. 1. In particular, a $90^0$ rotation in either direction is envisaged with the rotation axis passing through a center point of the ASG and the (possibly single) fixed focal spot FS1 of the X-ray source. Instead or in addition to a rotation, the ASG may be tilted by a certain angle that may be measured relative to the optical axis that passes through the center point of the ASG and the focal spot FS1. Instead or in addition of moving G0 or G2 to reduce the fringe pattern as discussed in the previous embodiment, it is the ASG that may be so moved.

For DAX/$\phi$ imaging, the mutual alignment of, in particular, the gratings G1, G2, G0 and the ASG, are essential for good object image acquisition quality. The proper alignment and focusing of these components may be cumbersome, time-consuming and not always easily achievable. Against this backdrop, having the control logic CL disturb either one of these components may be undesirable because the delicate refocusing or readjustment work may need to be re-done when switching back into image acquisition mode.

To address this problem, it is proposed in the alternative to have, over and above the described components, the facilitator SMF arranged as a separate, dedicated component arrangeable on demand in the beam between the patient and the detector or between source XS and patient. That is, rather than using as a scattering measurement facilitator existing hardware in a DAX/$\phi$ imaging setup, it is proposed to introduce a dedicated further component shown as component B into the beam when operating in scattering measurement mode. This component B is arranged to partially block out the x-radiation and can be manufactured similar to the gratings or the ASG with radiation blocking lamella or a plate with apertures, or other. The partial radiation blocking body B may be completely removed by an actuator eg, by sliding out of the beam for object acquisition mode and is slid back into the beam when the image system operates in scattering measuring mode.

In general, the control logic CL may be implemented by one or more computing units PU. The control logic may be fully integrated into the imaging system or may be arranged as one or more micro controllers but may also be arranged remotely at a remote one or more computing systems PU. The control logic CL may interact with the imaging system but components of the imaging system wirelessly or in a wired connection. The control logic CL may be arranged in software or hardware or partly in both. The control logic may be implemented as one or more microcontrollers, integrated into the imaging system IS.

Each one of the above described scattering measurement embodiments can be used singly on its own, or in combination or in any sub-combination.

It has been observed that the scattering management principles described herein yield very good contrast in DAX imagery. It is believed that is at least partly due to the fact that the proposed scattering reduction mechanism provides just the right amount of tolerance that is needed for DAX imaging. Too effective a scattering removal may actually hinder DAX imaging objectives as the rigorous removal of all scattering may run the risk of removing DAX effects altogether which then results in low DAX contrast. The proposed scattering management schemes described herein strike the right balance. Because of the switching between the two modes, two separate and/or dedicated measurement procedures are defined. In each procedure, the whole detector sensitive surface of the detector is at disposable for the respective measurement in embodiments. Operating in such dedicated/separate modes may also facilitate achieving the right scattering correction, particularly suited for the purpose of DAX imaging as mainly envisaged herein.

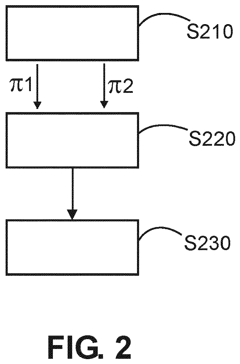

Reference is now made to the flow chart of Fig. 2 in which steps of a computer-implemented method for DAX/$\phi$ with scattering management is illustrated. It will be understood however that the method steps described in the following constitute a teaching in their own right and are not necessarily tied to the specific architecture shown in Fig. 1.

At step S210, an X-ray imaging apparatus XI is operated in two modes in sequence, first in one mode then in the other mode. The two modes are referred to herein as image acquisition mode and scattering measurement mode. The x-radiation receivable at the detector of the imaging system comprises a higher proportion of scattering when in scattering measurement mode as compared to scattering radiation receivable when in object acquisition mode. Step S210 results in two sets of measurement data $\pi1$ and $\pi2$, with $\pi2$ encoding a higher proportion of scattering radiation.

In particular, only, or predominantly, scattering radiation is measured in this second data set $\pi 2$, whilst set $\pi 1$ encodes primary radiation, with no or a lower fraction of secondary radiation.

In step S220, the scattering measurement data $\pi 2$ obtained in scattering measurement mode and the data $\pi 1$ obtained in image acquisition mode are used to generate scattering corrected imagery. This can be done for instance by simply dividing or subtracting the scattering measurement data from the image measurement data and to obtain difference image data and to then perform a phase retrieval operation based on the difference data to obtain the scattering corrected DAX- or $\phi$-imagery. Alternatively, a phase retrieval is performed first based on the data $\pi 1$ obtained in image acquisition mode to obtain preliminary DAX- or $\phi$-imagery. The preliminary DAX- or $\phi$-imagery is then post-processed based on the scattering measurement data $\pi 2$ to obtain scattering corrected DAX- or $\phi$-imagery. Alternatively, both data sets are processed in common in the phase retrieval to obtain the scattering corrected DAX- or $\phi$-imagery. Other scattering correction methods that are based on the scattering measurement data $\pi 2$ are also envisaged herein.

The scattering measurement data $\pi 2$ may be used directly as measured or may be pre-processed first, eg by filtering or other. In embodiments, a scattering simulation is used based on the measured data $\pi 2$.

In an optional step S230, the scattering corrected imagery is displayed on a display device or is otherwise made available.

One or more features disclosed herein may be configured or implemented as/with circuitry encoded within a computer-readable medium, and/or combinations thereof. Circuitry may include discrete and/or integrated circuitry, application specific integrated circuitry (ASIC), a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. An X-ray imaging system (XI) configured for phase contrast and/or dark-field imaging, comprising: an imaging apparatus, the imaging apparatus comprising

   - an X-ray source (XS) operable to cause X-radiation to emanate from a focal spot (SF) of the source (XS), and

   an X-ray sensitive detector (D) operable to detect the X-radiation after interaction of said X-radiation with an object to be imaged, if present, between the X-ray source and the detector (D); wherein the X-ray imaging system further comprises:

   - a control logic (CL) operable to cause the X-ray imaging apparatus to operate in any one of two modes, an object image acquisition mode and a scattering measurement mode, wherein, when in scattering measurement mode, the X-radiation receivable at the detector comprises a higher proportion of scattering radiation than in X-radiation receivable when the system is in object image acquisition mode, and
   - a scattering corrector (SC) operable, based on the measured scattering data, to reduce, or to facilitate reducing, or to prevent, a scattering artifact in an object image generatable from data acquired in image acquisition mode.

2. System of claim 1, comprising a scattering measurement facilitator (SMF) arranged between the X-ray source (SX) and the detector (D), at least a part or parts of the scattering measurement facilitator (SMF) capable of blocking X-radiation, the control logic causing, when the system is in scattering measurement mode, a change of pose of the scattering measurement facilitator relative to a pose of the scattering measurement facilitator when the system is in object image acquisition mode, so that at least the said part or parts block(s) at least some of the X-radiation.

3. System of claim 1 or 2, wherein the scattering measurement facilitator (SMF) comprises any one or more of: i) an anti-scatter grid (ASG), ii) at least a part of an interferometer (G0,G1,G2), iii) a code aperture plate.

4. System of any one of the preceding claims, where a direction of the X-radiation is changeable through a source interface (SF), wherein, the control logic (CL) causes through said interface (SF) X-radiation to emanate along a first direction when the system is in object image acquisition mode and cause the X-radiation to emanate along a second direction, different from the first direction, when the system is in scattering measurement mode.

5. System of claim 3, wherein the scattering measurement facilitator (SMF) comprises at least a part of the interferometer (G0,G1,G2), the control logic (CL), when in scattering measurement mode, causing the change of pose of the said interferometer, or the part thereof, so as to reduce a fringe pattern detectable at the detector.

6. System of any one of the previous claims, wherein the X-ray imaging system is a full-view imaging system.

7. A method comprising the steps of:

   causing (S210) an X-ray imaging apparatus (XI) configured for phase contrast and/or dark-field imaging to operate in any one of two modes, an object image acquisition mode and a scattering measurement mode, wherein, when in scattering measurement mode, the X-radiation receivable at a detector (D) of the apparatus (XI) comprises a higher proportion of scattering radiation than in X-radiation receivable when the system is in object image acquisition mode; and
   based on the measured scattering data, reducing (S220), or facilitating reducing, or preventing, a scattering artifact in an object image generatable from data acquired in image acquisition mode.

8. A computer program element, which, when being executed by at least one processing unit (PU), is adapted to cause the processing unit (PU) to perform the method as per claim 7.

9. A computer readable medium having stored thereon the program element of claim 8.

**Patentansprüche**

1. Röntgenbildsystem (XI), das für Phasenkontrast- und/oder Dunkelfeld-Bildgebung konfiguriert ist, das Folgendes umfasst:

- eine Röntgenquelle (XS), die so betrieben werden kann, dass sie Röntgenstrahlung aus einem Brennpunkt (SF) der Quelle (XS) ausstrahlt, und

einen röntgenstrahlenempfindlichen Detektor (D), der die Röntgenstrahlung nach

der Wechselwirkung der Röntgenstrahlung mit einem abzubildenden Objekt, falls vorhanden, zwischen der Röntgenquelle und dem Detektor (D) detektieren kann; wobei das Röntgenbildsystem ferner Folgendes umfasst:

- eine Steuerlogik (CL), die so betrieben werden kann, dass sie die Röntgenbild-Apparat veranlasst, in einem von zwei Modi zu arbeiten, einem Objektbilderfassungsmodus und einem Streuungsmessmodus, wobei im Streuungsmessmodus die am Detektor empfangbare Röntgenstrahlung einen höheren Anteil an Streustrahlung umfasst als die Röntgenstrahlung, die empfangen wird, wenn sich das System im Objektbilderfassungsmodus befindet, und

- einen Streuungskorrektor (SC), der auf der Grundlage der gemessenen Streuungsdaten betreibbar ist, um ein Streuungsartefakt in einem Objektbild, das aus den im Bilderfassungsmodus erfassten Daten erzeugt werden kann, zu reduzieren oder die Reduzierung zu erleichtern oder zu verhindern.

2. System nach Anspruch 1, das einen Streumessungserleichterer (SMF) umfasst, der zwischen der Röntgenquelle (SX) und dem Detektor (D) angeordnet ist, wobei mindestens ein Teil oder Teile des Streumessungserleichters (SMF) in der Lage sind, Röntgenstrahlung zu blockieren, wobei die Steuerlogik Folgendes bewirkt, wenn sich das System im Streuungsmessungsmodus befindet, eine Änderung der Position des Streuungsmessungserleichters relativ zu einer Position des Streuungsmessungserleichters, wenn sich das System im Objektbilderfassungsmodus befindet, so dass zumindest der Teil oder die Teile mindestens einen Teil der Röntgenstrahlung blockieren.

3. System nach Anspruch 1 oder 2, wobei der Streumessungsunterstützer (SMF) eines oder mehrere der folgenden Elemente umfasst: i) ein Antistreugitter (ASG), ii) mindestens einen Teil eines Interferometers (G0, G1, G2), iii) eine Code-Aperturplatte.

4. System nach einem der vorhergehenden Ansprüche, bei dem eine Richtung der Röntgenstrahlung durch eine Quellenschnittstelle (SF) veränderbar ist, wobei die Steuerlogik (CL) durch die Schnittstelle (SF) bewirkt, dass die Röntgenstrahlung entlang einer ersten Richtung ausgestrahlt wird, wenn sich das System im Objektbilderfassungsmodus befindet, und bewirkt, dass die Röntgenstrahlung entlang einer zweiten, sich von der ersten Richtung unterscheidenden Richtung ausgestrahlt wird, wenn sich das System im Streuungsmessmodus befindet.

5. System nach Anspruch 3, bei dem der Streumessungserleichterer (SMF) mindestens einen Teil des Interferometers (G0, G1, G2) umfasst, wobei die Steuerlogik (CL) im Streumessungsmodus die Änderung der Lage des Interferometers oder des Teils davon bewirkt, um ein am Detektor nachweisbares Streifenmuster zu reduzieren.

6. System nach einem der vorhergehenden Ansprüche, wobei das Röntgenbildsystem ein Vollsicht-Bildsystem ist.

7. Ein Verfahren, das die folgenden Schritte umfasst:

Veranlassen (S210) eines für die Phasenkontrast- und/oder Dunkelfeldabbildung konfigurierten Röntgenbild-Apparats (XI), in einem von zwei Modi zu arbeiten, einem Objektbilderfassungsmodus und einem Streuungsmessmodus, wobei im Streuungsmessmodus die an einem Detektor (D) des Apparats (XI) empfangbare Röntgenstrahlung einen höheren Anteil an Streustrahlung umfasst als in der Röntgenstrahlung, die empfangen werden kann, wenn sich das System im Objektbilderfassungsmodus befindet; und basierend auf den gemessenen Streudaten, ein Streuungsartefakt in einem Objektbild, das aus den im Bilderfassungsmodus erfassten Daten erzeugt werden kann, zu reduzieren (S220), zu erleichtern oder zu verhindern.

8. Computerprogrammelement, das, wenn es von mindestens einer Verarbeitungseinheit (PU) ausgeführt wird, geeignet ist, die Verarbeitungseinheit (PU) zu veranlassen, das Verfahren nach Anspruch 7 durchzuführen.

9. Computerlesbares Medium, auf dem das Programmelement nach Anspruch 8 gespeichert ist.

**Revendications**

1. Système d'imagerie à rayons X (XI) configuré pour une imagerie à contraste de phase et/ou en champ sombre, comprenant: un appareil d'imagerie, l'appareil d'imagerie comprenant

- une source de rayons X (XS) utilisable pour faire en sorte que des rayons X émanent d'un point focal (SF) de la source (XS), et

un détecteur sensible aux rayons X (D) pouvant fonctionner pour détecter le rayonnement X après interaction dudit rayonnement X avec un objet à localiser, s'il est présent, entre la source de rayons X et le détecteur (D); dans lequel le système d'imagerie à rayons X comprend en outre:

- une logique de commande (CL) pouvant être mise en œuvre pour amener l'appareil d'imagerie à rayons X à fonctionner dans l'un quelconque de deux modes, un mode d'acquisition d'image d'objet et un mode de mesure de diffusion, dans lequel, lorsqu'il est en mode de mesure de diffusion, le rayonnement X pouvant être reçu au niveau du détecteur comprend une proportion plus élevée de rayonnement de diffusion que dans le rayonnement X pouvant être reçu lorsque le système est en mode d'acquisition d'image d'objet, et

- un correcteur de diffusion (SC) pouvant fonctionner, sur la base des données de diffusion mesurées, pour réduire, ou pour faciliter la réduction, ou pour empêcher, un artefact de diffusion dans une image d'objet pouvant être générée à partir de données acquises en mode d'acquisition d'image.

2. Système selon la revendication 1, comprenant un facilitateur de mesure de diffusion (SMF) disposé entre la source de rayons X (SX) et le détecteur (D), au moins une partie ou des parties du facilitateur de mesure de diffusion (SMF) étant capables de bloquer le rayonnement X, la logique de commande provoquant, lorsque le système est en mode de mesure de diffusion, un changement de pose du facilitateur de mesure de diffusion par rapport à une pose du facilitateur de mesure de diffusion lorsque le système est en mode d'acquisition d'image d'objet, de sorte qu'au moins ladite partie ou lesdites parties bloquent au moins une partie du rayonnement X.

3. Système selon la revendication 1 ou 2, dans lequel le facilitateur de mesure de diffusion (SMF) comprend un ou plusieurs des éléments suivants: i) une grille antidiffusante (ASG), ii) au moins une partie d'un interféromètre (G0, G1, G2), iii) une plaque d'ouverture de code.

4. Système selon l'une quelconque des revendications précédentes, dans lequel une direction du rayonnement X peut être modifiée par l'intermédiaire d'une interface de source (SF), dans lequel la logique de commande (CL) fait en sorte que, par l'intermédiaire de ladite interface (SF), un rayonnement X émane le long d'une première direction lorsque le système est en mode d'acquisition d'image d'objet et fait en sorte que le rayonnement X émane le long d'une deuxième direction, différente de la première direction, lorsque le système est en mode de mesure de diffusion.

5. Système selon la revendication 3, dans lequel le facilitateur de mesure de diffusion (SMF) comprend au moins une partie de l'interféromètre (G0, G1, G2), la logique de commande (CL), lorsqu'elle est en mode de mesure de diffusion, provoquant le changement de pose dudit interféromètre, ou de la partie de celui-ci, de façon à réduire un motif de franges détectable au niveau du détecteur.

6. Système selon l'une quelconque des revendications précédentes, dans lequel le système d'imagerie à rayons X est un système d'imagerie à vue complète.

7. Procédé comprenant les étapes consistant à:

faire en sorte (S210) qu'un appareil d'imagerie à rayons X (XI) configuré pour une imagerie à contraste de phase et/ou en champ sombre fonctionne dans l'un quelconque de deux modes, un mode d'acquisition d'image objet et un mode de mesure de diffusion, dans lequel, lorsqu'il est en mode de mesure de diffusion, le rayonnement X pouvant être reçu au niveau d'un détecteur (D) de l'appareil (XI) comprend une proportion plus élevée de rayonnement de diffusion que dans le rayonnement X pouvant être reçu lorsque le système est en mode d'acquisition d'image objet; et

sur la base des données de diffusion mesurées, réduire (S220), ou faciliter la réduction, ou empêcher, un artefact de diffusion dans une image d'objet pouvant être générée à partir de données acquises en mode d'acquisition d'image.

8. Élément de programme informatique qui, lorsqu'il est exécuté par au moins une unité de traitement (PU), est adapté pour amener l'unité de traitement (PU) à exécuter le procédé selon la revendication 7.

9. Support lisible par ordinateur sur lequel est stocké l'élément de programme de la revendication 8.

**FIG. 1**

$\pi 1$     $\pi 2$

FIG. 2

**EP 3 911 236 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017284948 A1

**Non-patent literature cited in the description**

- **A. YAROSHENKO et al.** Pulmonary Emphysema Diagnosis with a Preclinical Small-Animal X-ray Dark-Field Scatter-Contrast Scanner. *Radiology,* November 2013, vol. 269 (2

- **PFEIFFER et al.** Hard-X-ray dark-field imaging using a grating interferometer. *Nature Materials,* 2008, vol. 7, 134-137